# EUROPEAN PATENT SPECIFICATION

(11) **EP 1 996 943 B1**
(45) Date of publication and mention of the grant of the patent: **25.06.2014**
(21) Application number: 07711809.9
(22) Date of filing: 07.03.2007
(51) Int. Cl.: G01N 33/543, G01N 33/68

(54) **ANTI-DRUG ANTIBODY ASSAY**
ASSAY FÜR ANTIKÖRPER GEGEN ARZNEISTOFF
ANALYSE D'ANTICORPS ANTI-MÉDICAMENT

(30) Priority: 09.03.2006 EP 06004806
(43) Date of publication of application: 03.12.2008
(73) Proprietor: F.Hoffmann-La Roche AG, 4070 Basel (CH)
(72) Inventor: HOESEL, Wolfgang, 82327 Tutzing (DE); STUBENRAUCH, Kay-Gunnar, 82377 Penzberg (DE); VOGEL, Rudolf, 82362 Weilheim (DE)
(74) Representative: Burger, Alexander
(86) International application number: PCT/EP2007/001935
(87) International publication number: WO 2007/101661

(56) References cited:
- BOURDAGE ET AL: "Effect of double antigen bridging immunoassay format on antigen coating concentration dependence and implications for designing immunogenicity assays for monoclonal antibodies" JOURNAL OF PHARMACEUTICAL AND BIOMEDICAL ANALYSIS, NEW YORK, NY, US, vol. 39, no. 3-4, 15 September 2005 (2005-09-15), pages 685-690, XP005039077 ISSN: 0731-7085
- THURMOND L M ET AL: "A kinetic enzyme immunoassay for the quantitation of antibodies to a humanized monoclonal antibody in human serum" JOURNAL OF PHARMACEUTICAL AND BIOMEDICAL ANALYSIS, vol. 16, no. 8, April 1998 (1998-04), pages 1317-1328, XP002389028 ISSN: 0731-7085
- BAERT F ET AL: "INFLUENCE OF IMMUNOGENICITY ON THE LONG-TERM EFFICACY OF INFLIXIMAB IN CROHN'S DISEASE" 13 February 2003 (2003-02-13), NEW ENGLAND JOURNAL OF MEDICINE, MASSACHUSETTS MEDICAL SOCIETY, BOSTON, MA, US, PAGE(S) 601-608 , XP009037271 ISSN: 1533-4406 page 602 - page 603
- HOESEL W ET AL: "Development and evaluation of a new ELISA for the detection and quantification of antierythropoietin antibodies in human sera" JOURNAL OF IMMUNOLOGICAL METHODS, ELSEVIER SCIENCE PUBLISHERS B.V.,AMSTERDAM, NL, vol. 294, no. 1-2, November 2004 (2004-11), pages 101-110, XP004679759 ISSN: 0022-1759

## Description

The invention comprises a method for the determination of anti-drug antibodies and kits for the use of such assays.

### Background of the Invention

Standard solid-phase immunoassays with monoclonal antibodies involve the formation of a complex between an antibody adsorbed/immobilized on a solid phase (capture antibody), the antigen, and an antibody to another epitope of the antigen conjugated with an enzyme (tracer antibody). Thus, a sandwich is formed: solid phase-capture antibody-antigen-tracer antibody. In the reaction catalyzed by the sandwich, the activity of the antibody-conjugated enzyme is proportional to the antigen concentration in the incubation medium. The standard sandwich method is also called double antigen bridging immunoassay because capture and tracer antibodies bind to different epitopes of the antigen. Hoesel, W., et al., in J. Immunol. Methods 294 (2004) 101-110, report an anti-EPO double antigen bridging assay whereby a mixture of immobilized rhEPO coupled to amino groups and to carbohydrate groups was used. Immunoassays such as the double antigen bridging ELISA are common assay types in the investigation of an immunogenic answer of a patient to an antibody drug. Mire-Sluis, A.R., et al., J. Immunol. Methods 289 (2004) 1-16, summarize the recommendations for the design and optimization of immunoassays using detection of host antibodies against biotechnology products. According to Mire-Sluis et al. the well-known anti-drug antibody assay formats show considerable disadvantages. Anti-drug antibody assays are mentioned, for example, in WO 2005/045058 and WO 90/006515. Anti-idiotypic antibody assays are mentioned, for example, in US 5,219,730; WO 87/002778; EP 0 139 389; and EP 0 170 302. Wadhwa, M., et al., in J. Immunol. Methods 278 (2003) 1-17, report strategies for the detection, measurement and characterization of unwanted antibodies induced by therapeutic biologicals.

Bourdage, J.S. et al. in J.Pharm. Biomed. Anal_39_(2005)_685-690 reported an effect of double antigen bridging immunoassay format on antigen coating concentration dependence and implications for designing immunogenicity assays for monoclonal antibodies. A kinetic enzyme immunoassay for the quantitation of antibodies to a humanized monoclonal antibody in human serum is reported in Thurmond, L., M. et al. (J. Pharm. Biomed. Anal_16_(1998)_1317-1328). Baert, F. et al. _N. Engl. J. Med_348_(2003)_601-608 report on the influence of immunogenicity on the long-term efficacy of infliximab in Crohn's Disease.

### Summary of the Invention

The invention provides methods and means for the immunological determination of an antibody against a drug antibody in a sample using a double antigen bridging immunoassay.

The invention provides a method for the immunological determination of an antibody against a drug antibody in a sample using a double antigen bridging immunoassay comprising a capture drug antibody and a tracer drug antibody, characterized in that the capture drug antibody is a mixture of said drug antibody comprising at least two of said drug antibodies that differ in the antibody site at which they are conjugated to the solid phase, and the tracer drug antibody is a mixture of said drug antibody comprising at least two of said drug antibodies that differ in the antibody site at which they are conjugated to the detectable label.

Preferably conjugation of the drug antibody to its conjugation partner is performed by chemically binding via N-terminal and/or ε-amino groups (lysine), ε-amino groups of different lysines, carboxy-, sulfhydryl-, hydroxyl- and/or phenolic functional groups of the amino acid backbone of the drug antibody and/or sugar alcohol groups of the carbohydrate structure of the drug antibody.

Preferably the capture drug antibody mixture comprises the drug antibody conjugated via an amino group and via a carbohydrate structure to their conjugation partner.

Preferably the capture drug antibody mixture and/or the tracer drug antibody mixture comprise the drug antibody conjugated via at least two different amino groups to their conjugation partner. Such coupling via different amino groups can be performed by acylation of a part of the ε-amino groups with chemical protecting agents, e.g. by citraconylation, in a first step. In a second step conjugation is performed via the remaining amino groups. Subsequently citraconylation is removed and the drug antibody is conjugated to the conjugation partner via remaining free amino groups, i.e. the drug antibody obtained is conjugated to the conjugation partner via amino groups that have not been proteced by citraconylation.

Suitable chemical protecting agents form bonds at unprotected side chain amines and are less stable than and different from those bonds at the N-terminus. Many such chemical protecting agents are known (see for example European Patent Application EP 0 651 761). Preferred chemical protecting agents include cyclic dicarboxylic acid anhydrides like maleic or citraconylic acid anhydrides.

Preferably the capture drug antibody is conjugated to the solid phase by passive adsorption and therefore is conjugated to the solid phase at at least two different antibody sites. Passive adsorption is, e. g., described by Butler, J.E., in "Solid Phases in Immunoassay" 205-225; Diamandis, E.P., and Christopoulos, T.K. (Editors): Immunoassays (1996) Academic Press San Diego.

Preferably the tracer drug antibody mixture comprises the drug antibody conjugated via an amino group and via a carbohydrate structure to its conjugation partner.

Preferably the ratio of capture drug antibody to tracer drug antibody is 1:10 to 50:1 (ratio means ratio of antibody molecules irrespective of the molecular weight of the conjugates which can be different).

Preferably the ratio of amino conjugated drug antibody (either tracer or capture drug antibody) to carbohydrate conjugated drug antibody (either tracer or capture drug antibody) in such a mixture is 1:10 to 10:1 (ratio means ratio of antibody molecules irrespective of the molecular weight of the conjugates which can be different).

In a preferred embodiment of the invention, the capture drug antibody is conjugated (immobilized) via a specific binding pair. Such a binding pair (first component/second component) is, for example, streptavidin or avidin/biotin, antibody/antigen (see, for example, Hermanson, G.T., et al., Bioconjugate Techniques, Academic Press, 1996), lectin/polysaccharide, steroid/steroid binding protein, hormone/hormone receptor, enzyme/substrate, IgG/Protein A and/or G, etc. Preferably, the capture drug antibody is conjugated to biotin and immobilization is performed via immobilized avidin or streptavidin.

In a preferred embodiment of the invention, the tracer drug antibody is conjugated to a detectable label, preferably conjugated via a specific binding pair. Such a binding pair (first component/second component) is, for example, streptavidin or avidin/biotin, antibody/antigen (see, for example, Hermanson, G.T., et al., Bioconjugate Techniques, Academic Press, 1996), lectin/polysaccharide, steroid/steroid binding protein, hormone/hormone receptor, enzyme/substrate, IgG/Protein A and/or G, etc. Preferably, the tracer drug antibody is conjugated via digoxigenin and an antibody against digoxigenin to the detectable label. Alternatively the tracer drug antibody is conjugated to an electrochemiluminescent label, like a ruthenium bispyridyl complex.

### Detailed Description of the Invention

The term "drug antibody" according to the invention denotes an antibody which can be administered to an individual, so that a sample of said individual is suspected to comprise said drug antibody after administration. Within one assay performed according to the invention the drug antibody, the capture drug antibody and the tracer drug antibody comprise the "same" antibody molecule, e.g. recombinantly produced with the same expression vector and comprising the same amino acid sequence. Drug antibodies (therapeutic monoclonal antibodies) are being used widely for the treatment of various diseases such as oncological diseases (e.g. hematological and solid malignancies including non-Hodgkin's lymphoma, breast cancer, and colorectal cancer). Such antibodies are described, for example, by Levene, A.P., et al., Journal of the Royal Society of Medicine 98 (2005) 146-152. Such antibodies are, for instance, antibodies against CD20, CD22, HLA-DR, CD33, CD52, EGFR, G250, GD3, HER2, PSMA, CD56, VEGF, VEGF2, CEA, Levis Y antigen, IL-6 receptor or IGF-1 receptor. Therapeutic antibodies are also described by Groner, B., et al., Curr. Mol. Med. 4 (2004) 539-547; and Harris, M., Lancet Oncol. 5 (2004) 292-302.

An example (preferably monoclonal) antibody is an antibody against IL-6 receptor (mAB IL-6R). Such an antibody is for example described by Mihara et al., Clin. Immunol. 98 (2001) 319-326; Nishimoto, N., et al, Blood 106 (2005) 2627-2632, in Illei, G.G. et al. 62(2) (2010) 542-552 (clinical trial NCT00046774), or in WO 2004/096274.

An example (preferably monoclonal) antibody is an antibody against IGF-1 receptor (mAB IGF-1R). Such an antibody is for example described in WO 2004/087756, or in WO 2005/005635.

Anti-drug antibodies are antibodies, which are directed against any region of the drug antibody, like the variable region, the constant region or the glycostructure of the drug antibody. Such anti-drug antibodies may occur during antibody therapy as an immunogenic reaction of a patient (see Pan, Y., et al., FASEB J. 9 (1995) 43-49).

Monoclonal antibodies contain as proteins a number of reactive side chains. Such reactive chemical groups of antibodies are, for example, amino groups (lysines, alpha-amino groups), thiol groups (cystines, cysteine, and methionine), carboxylic acid groups (aspartic acid, glutamic acid), and sugar-alcoholic groups.

Solid supports for the immunoassays according to the invention are widely described in the state of the art (see, e.g., Butler, J.E., Methods 22 (2000) 4-23).

The principles of different immunoassays are described, for example, by Hage, D.S., in Anal. Chem. 71 (1999) 294R-304R. Lu, B., et al., Analyst 121 (1996) 29R-32R, report the orientated immobilization of antibodies for the use in immunoassays. Avidin-biotin-mediated immunoassays are reported, for example, by Wilchek, M., and Bayer, E.A., Methods Enzymol. 184 (1990) 467-469.

Monoclonal antibodies and their constant domains contain as proteins a number of reactive side chains for coupling to a binding partner like a surface, a protein, a polymer, such as PEG, Cellulose or Polystyrol, an enzyme, or a member of a binding pair. Chemical reactive groups of antibodies are, for example, amino groups (lysines, alpha-amino groups), thiol groups (cystines, cysteines, and methionines), carboxylic acid groups (aspartic acids, glutamic acids), and sugar-alcoholic groups. Such methods are e.g. described by Aslam M., and Dent A., Bioconjugation, MacMillan Ref. Ltd. 1998, pp. 50-100.

One of the most common reactive groups of proteins is the aliphatic ε-amine of the amino acid lysine. In general, nearly all antibodies contain abundant lysine. Lysine amines are reasonably good nucleophiles above pH 8.0 (pKa = 9.18) and therefore react easily and cleanly with a variety of reagents to form stable bonds.

Another common reactive group in antibodies is the thiol residue from the sulfur-containing amino acid cystine and its reduction product cysteine (or half cystine). Cysteine contains a free thiol group, which is more nucleophilic than amines and is generally the most reactive functional group in a protein. Thiols are generally reactive at neutral pH, and therefore can be coupled to other molecules selectively in the presence of amines.

Since free sulfhydryl groups are relatively reactive, proteins with these groups often exist with them in their oxidized form as disulfide groups or disulfide bonds. Immunoglobulin M is an example of a disulfide-linked pentamer, while the subunits of Immunoglobulin G are bonded by internal disulfide bridges. In such proteins, reduction of the disulfide bonds with a reagent such as dithiothreitol (DTT) is required to generate the reactive free thiol. However, this method also splits the chain linkage in these antibodies and reassembly of the chains to allow proper folding may not be possible. In addition to cystine and cysteine, some proteins also have the amino acid methionine which is containing sulfur in a thioether linkage. Selective modification of methionine is generally difficult to achieve and is seldom used as a method of attaching drugs and other molecules to antibodies. The literature reports the use of several thiolating crosslinking reagents such as Traut's reagent (2-iminothiolane), succinimidyl (acetylthio) acetate (SATA), and sulfosuccinimidyl 6-[3-(2-pyridyldithio)propionamido]hexanoate (Sulfo-LC-SPDP) to provide efficient ways of introducing multiple sulfhydryl groups via reactive amino groups.

Bioconjugation chemistry is the joining of biomolecules to other biomolecules, small molecules, and polymers by chemical or biological means. This includes the conjugation of antibodies and their fragments, nucleic acids and their analogs, and liposomal components (or other biologically active molecules) with each other or with any molecular group that adds useful properties. These molecular groups include radionuclides, drugs, toxins, enzymes, metal chelates, fluorophores, haptens, and others.

Another common reactive group in antibodies are carboxylic acids (aspartic acid, glutamic acid). Proteins contain carboxylic acid groups at the C-terminal position and within the side chains of aspartic acid and glutamic acid. The relatively low reactivity of carboxylic acids in water usually makes it difficult to use these groups to selectively modify proteins and other biomolecules. When this is done, the carboxylic acid group is usually converted to a reactive ester by the use of a water-soluble carbodiimide and reacted with a nucleophilic reagent such as an amine, hydrazide, or hydrazine. The amine-containing reagent should be weakly basic in order to react selectively with the activated carboxylic acid in the presence of other amines on the protein. Protein crosslinking can occur when the pH is raised above 8.0.

Sodium periodate can be used to oxidize the alcohol part of a sugar within a carbohydrate moiety to an aldehyde. Each aldehyde group can be reacted with an amine, hydrazide, or hydrazine as described for carboxylic acids. Since the carbohydrate moiety is predominantly found on the crystallizable fragment (Fc) region of an antibody, conjugation can be achieved through site-directed modification of the carbohydrate away from the antigen-binding site.

Amine-reactive reagents react primarily with lysines and the α-amino groups of proteins. Reactive esters, particularly N-hydroxy-succinimide (NHS) esters, are among the most commonly employed reagents for modification of amine groups. The optimum pH for reaction in an aqueous environment is pH 8.0 to 9.0. Isothiocyanates are amine-modification reagents and form thiourea bonds with proteins. They react with protein amines in aqueous solution (optimally at pH 9.0 to 9.5). Aldehydes react under mild aqueous conditions with aliphatic and aromatic amines, hydrazines, and hydrazides to form an imine intermediate (Schiffs base). A Schiffs base can be selectively reduced with mild or strong reducing agents (such as sodium borohydride or sodium cyanoborohydride) to derive a stable alkyl amine bond.

Other reagents that have been used to modify amines are acid anhydrides. For example, diethylenetriaminepentaacetic anhydride (DTPA) is a bifunctional chelating agent that contains two amine-reactive anhydride groups. It can react with N-terminal and ε-amine groups of proteins to form amide linkages. The anhydride rings open to create multivalent, metal-chelating arms able to bind tightly to metals in a coordination complex.

Thiol-reactive reagents are those that will couple to thiol groups on proteins, forming thioether-coupled products. These reagents react rapidly at slight acidic to neutral pH and therefore can be reacted selectively in the presence of amine groups.

Haloacetyl derivatives, e.g. iodoacetamides, form thioether bonds and are reagents for thiol modification. In antibodies, the reaction takes place at cysteine groups that are either intrinsically present or that result from the reduction of cystine's disulfides at various positions of the antibody.

Further useful reagents are maleimides. The reaction of maleimides with thiol-reactive reagents is essentially the same as with iodoacetamides. Maleimides react rapidly at slight acidic to neutral pH.

Amines, hydrazides, and hydrazines are aldehyde and carboxylic acid-reactive reagents (formation of amide, hydrazone, or alkyl amine bonds). Amines, hydrazides, and hydrazines can be coupled to carboxylic acids of proteins after the activation of the carboxyl group by a water-soluble carbodiimide. The amine-containing reagent must be weakly basic so that it reacts selectively with the carbodiimide-activated protein in the presence of the more highly basic ε-amines of lysine to form a stable amide bond.

Amines, hydrazides, and hydrazines can also react with aldehyde groups, which can be generated on antibodies by periodate oxidation of the carbohydrate residues on the antibody. In this scenario, a Schiff's base intermediate is formed, which can be reduced to an alkyl amine through the reduction of the intermediate with sodium cyanoborohydride (mild and selective) or sodium borohydride (strong) water-soluble reducing agents.

The term "sample" includes, but is not limited to, any quantity of a substance from a living thing or formerly living thing. Such living things include, but are not limited to, humans, mice, monkeys, rats, rabbits, and other animals. Such substances include, but are not limited to, whole blood, serum, or plasma from an individual, which are the most widely used sources of sample in clinical routine.

The term "solid phase" means a non-fluid substance, and includes particles (including microparticles and beads) made from materials such as polymer, metal (paramagnetic, ferromagnetic particles), glass, and ceramic; gel substances such as silica, alumina, and polymer gels; capillaries, which may be made of polymer, metal, glass, and/or ceramic; zeolites and other porous substances; electrodes; microtiter plates; solid strips; and cuvettes, tubes or other spectrometer sample containers. A solid phase component of an assay is distinguished from inert solid surfaces with which the assay may be in contact in that a "solid phase" contains at least one moiety on its surface, which is intended to interact with the capture drug antibody. A solid phase may be a stationary component, such as a tube, strip, cuvette or microtiter plate, or may be non-stationary components, such as beads and microparticles. Microparticles can also be used as a solid phase for homogeneous assay formats. A variety of microparticles that allow either non-covalent or covalent attachment of proteins and other substances may be used. Such particles include polymer particles such as polystyrene and poly(methylmethacrylate); gold particles such as gold nanoparticles and gold colloids; and ceramic particles such as silica, glass, and metal oxide particles. See for example Martin, C.R., et al., Analytical Chemistry-News & Features, May 1, 1998, 322A-327A, which is incorporated herein by reference.

Chromogens (fluorescent or luminescent groups and dyes), enzymes, NMR-active groups or metal particles, haptens, e.g. digoxigenin, are examples of detectable labels. The detectable label can also be a photoactivatable crosslinking group, e.g. an azido or an azirine. group. Metal chelates which can be detected by electrochemoluminescence are also preferred signal-emitting groups, with particular preference being given to ruthenium chelates, e.g. a ruthenium (bispyridyl)₃²⁺ chelate. Suitable ruthenium labeling groups are described, for example, in EP 0 580 979, WO 90/05301, WO 90/11511, and WO 92/14138.

The invention provides a method for the immunological determination of an antibody against a drug antibody in a sample using a double antigen bridging immunoassay comprising a capture drug antibody and a tracer drug antibody, wherein the capture drug antibody is a mixture of the drug antibody comprising at least two of the drug antibodies that differ in the antibody site at which they are conjugated to the solid phase, and the tracer drug antibody is a mixture of the drug antibody comprising at least two of the drug antibodies that differ in the antibody site at which they are conjugated to the detectable label.

The capture drug antibody useful in a method according to the invention is conjugated to a solid phase. The conjugation is preferably performed by chemical binding via N-terminal and/or ε-amino groups (lysine), ε-amino groups of different lysines, carboxy-, sulfhydryl-, hydroxyl- and/or phenolic functional groups of the amino acid backbone of the drug antibody and/or sugar alcohol groups of the carbohydrate structure of the drug antibody. The capture drug antibody useful in a method according to the invention is a mixture of at least two drug antibodies conjugated to a solid phase, wherein said at least two drug antibodies conjugated to a solid phase differ in the site at which they are conjugated to the solid phase. For example, the mixture of at least two drug antibodies conjugated to a solid phase may comprise a drug antibody conjugated via an amino acid of the amino acid backbone to the solid phase and a drug antibody conjugated via a sugar alcohol group of a carbohydrate structure of the drug antibody to the solid phase. Also for example, the mixture of at least two drug antibodies conjugated to a solid phase may comprise drug antibodies conjugated to the solid phase via different amino acid residues of their amino acid backbone. The expression "different amino acid residue" denotes either two different kinds of amino acids, such as e.g. lysine and aspartic acid, or tyrosine and glutamic acid, or two numeral different amino acid residues of the amino acid backbone of the drug antibody. In the latter case the amino acid can be of the same kind or of different kind. The expressions "differ in the antibody site" and "site" denote a difference either in the kind of site, e.g. amino acid or sugar alcohol group, or in the number of the amino acid of the amino acid backbone at which the drug antibody is conjugated to the solid phase. The same applies vice versa to the tracer drug antibody useful in a method according to the invention.

The following examples and figures are provided to aid the understanding of the present invention, the true scope of which is set forth in the appended claims.

### Description of Figures

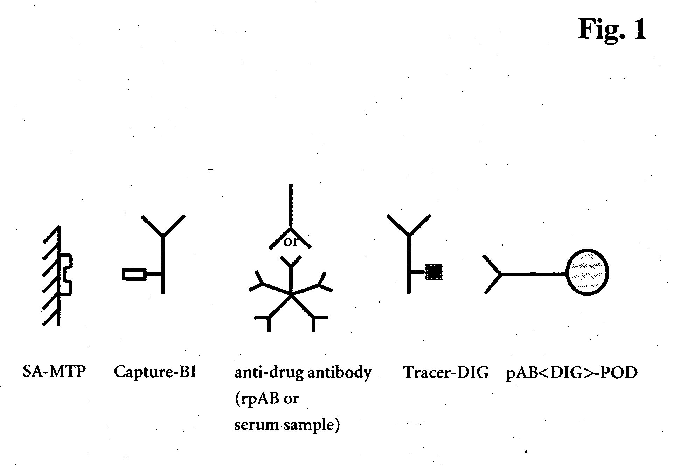

- **Figure 1**: Bridging assay for detection of anti-drug antibodies:
The biotinylated drug antibody (Capture-BI) is bound to a streptavidin-coated microtiter plate (SA-MTP). The anti-drug antibody bridges the capture drug antibody (Capture-BI; BI=biotinylated) with digoxigenin-labeled tracer drug antibody (Tracer-DIG; DIG=digoxinylated). The immobilized complex is detected by polyclonal anti-digoxigenin horse-radish peroxidase conjugate (pAB<DIG>-POD). Polyclonal rabbit anti-drug antibody (rpAB) is used as standard.
- **Figure 2**: Standard curve of bridging ELISA variant 1 using conjugates of example 1 and 4:
The optical densities (ODs) are given for the various concentration of rpAB as diluted in PBS-T-buffer (phosphate buffered saline, 0.05 Vol% Tween^{®} 20) with 5% human serum.
- **Figure 3**: Standard curve of bridging ELISA variant 2 using conjugates of example 2 and 5:
The optical densities (ODs) are given for the various concentration of rpAB as diluted in PBS-T-buffer with 5% human serum.
- **Figure 4**: Standard curve of bridging ELISA variant 3 using conjugates of example 3 and 6:
The optical densities (ODs) are given for the various concentration of rpAB as diluted in PBS-T-buffer with 5% human serum.
- **Figure 5**: Standard curve of bridging ELISA variant 4 using conjugates of examples 1, 3 and 4, 6:
The optical densities (ODs) are given for the various concentration of rpAB as diluted in PBS-T-buffer with 5% human serum.
- **Figure 6**: Standard curve of bridging ELISA variant 5 using conjugates of example 1, 2, 3 and 4, 5, 6:
The optical densities (ODs) are given for the various concentration of rpAB as diluted in PBS-T-buffer with 5% human serum.
- **Figure 7**: Standard curve of bridging ELISA variant 1 using passive adsorption for solid immobilization:
The optical densities (ODs) are given for the various concentration of rpAB as diluted in PBS-T-buffer with 5% human serum.

### Examples

### Example 1

### Biotinylation of antibody mAB IL-6R with D-biotinoyl-aminocaproic acid-N-hydroxysuccinimide ester

Antibody against IL-6 receptor (mAB IL-6R) has been dialyzed against buffer (100 mM potassium phosphate buffer (in the following denoted as K-PO₄), pH 8.5). Afterwards the solution was adjusted to a protein concentration of 10 mg/ml. D-biotinoyl-aminocaproic acid-N-hydroxysuccinimide ester was dissolved in DMSO and added to the antibody solution in a molar ratio of 1:5. After 60 minutes the reaction was stopped by adding L-lysine. The excess of the labeling reagent was removed by dialysis against 25 mM K-PO₄ supplemented with 150 mM NaCl, pH 7.5.

### Example 2

### Biotinylation of mAB IL-6R with D-biotinoyl-aminocaproic acid-N-hydroxysuccinimide ester after treatment with citraconic acid anhydride

mAB IL-6R has been dialyzed against 100 mM K-PO₄, pH 8.4. Afterwards the solution was adjusted to a protein concentration of 20 mg/ml. Citraconic acid anhydride was dissolved in DMSO and added to the antibody solution in a molar ratio of 1:5. After 120 minutes the reaction was stopped by chromatography on a column with Sephadex^{®} G25 equilibrated with 100 mM K-PO₄, pH 8.4. The antibody solution was adjusted to a protein concentration of about 4 mg/ml. D-biotinoyl-aminocaproic acid-N-hydroxysuccinimide ester was dissolved in DMSO and added to the antibody solution in a molar ratio of 1:5. The reaction was stopped after 60 minutes by adding L-lysine. The surplus of the labeling reagent was removed by dialysis against 200 mM sodium acetate buffer, pH 5.0. The antibody solution was transferred to a 25 mM K-PO₄ supplemented with 150 mM NaCl, pH 7.2, by chromatography on a column with Sephadex^{®} G25.

### Example 3

### Biotinylation of mAB IL-6R with biotin hydrazide

mAB IL-6R has been dialyzed against 100 mM sodium acetate buffer, pH 5.5. Afterwards the solution was adjusted to a protein concentration of 20 mg/ml. Sodium periodate was dissolved in 100 mM sodium acetate buffer, pH 5.5, and was added to the antibody solution to a final concentration of 10 mM. The reaction was stopped after 30 minutes by chromatography on a Sephadex^{®} G25 column equilibrated with 100 mM sodium acetate buffer, pH 5.5. The antibody solution was adjusted to a protein concentration of about 5 mg/ml. Biotin hydrazide was dissolved in DMSO and added to the antibody solution in a molar ratio of 1:50. The reaction was stopped after 120 minutes by adding sodium borohydride to a final concentration of 15 mM. After 30 minutes the antibody solution was dialyzed against 25 mM K-PO₄ supplemented with 150 mM NaCl, pH 7.2

### Example 4

### Digoxigenylation of mAB IL-6R with digoxigenin 3-O-methylcarbonyl-ε-aminocaproic acid-N-hydroxysuccinimide ester

mAB IL-6R has been dialyzed against digoxigenylation buffer (100 mM K-PO₄, pH 8.5). Afterwards the solution was adjusted to a protein concentration of 10 mg/ml. Digoxigenin 3-O-methylcarbonyl-ε-aminocaproic acid-N-hydroxysuccinimide ester was dissolved in DMSO and added to the antibody solution in a molar ratio of 1:5. After 60 minutes the reaction has been stopped by adding L-lysine. The surplus of labeling reagent was removed by dialysis against 25 mM K-PO₄ supplemented with 150 mM NaCl, pH 7.5.

### Example 5

### Digoxigenylation of mAB IL-6R with digoxigenin 3-O-methylcarbonyl-ε-aminocaproic acid-N-hydroxysuccinimide ester after treatment with citraconic acid anhydride

mAB IL-6R has been dialyzed against 100 mM K-PO₄, pH 8.4. Afterwards the solution was adjusted to a protein concentration of 20 mg/ml. Citraconic acid anhydride was dissolved in DMSO and added to the antibody solution in a molar ratio of 1:5. The reaction has been stopped after 120 minutes by chromatography on a column with Sephadex^{®} G25 equilibrated with 100 mM K-PO₄, pH 8.4. The antibody solution was adjusted to a protein concentration of about 4 mg/ml. Digoxigenin 3-O-methylcarbonyl-ε-aminocaproic acid-N-hydroxysuccinimide ester was dissolved in DMSO and added to the antibody solution in a molar ratio of 1:5. The reaction has been stopped after 60 minutes by adding L-lysine. The surplus of the labeling reagent was removed by dialysis against 200 mM sodium acetate buffer, pH 5.0. The antibody solution was transferred to a buffer with 25 mM K-PO₄ and 150 mM NaCl, pH 7.2, by chromatography on a column with Sephadex^{®} G25.

### Example 6

### Digoxigenylation of mAB IL-6R with digoxigenin-X-hydrazide

mAB IL-6R has been dialyzed against 100 mM sodium acetate buffer, pH 5.5. Afterwards the solution was adjusted to a protein concentration of 20 mg/ml. Sodium periodate was dissolved in 100 mM sodium acetate buffer, pH 5.5, and was added to the antibody solution to a final concentration of 10 mM. The reaction has been stopped after 30 minutes by chromatography on a Sephadex^{®} G25 column equilibrated with 100 mM sodium acetate buffer, pH 5.5. The antibody solution was adjusted to a protein concentration of about 5 mg/ml. Digoxigenin-X-hydrazide was dissolved in DMSO and added to the antibody solution in a molar ratio of 1:50. After 120 minutes the reaction has been stopped by adding sodium borohydride to a final concentration of 15 mM. After 30 minutes the antibody solution was dialyzed against 25 mM K-PO₄ supplemented with 150 mM NaCl, pH 7.2

### Example 7

### Bridging ELISA for detection of antibodies against mAB IL-6R

Biotinylated mAB IL-6R has been conjugated to (bound onto) the wells of a streptavidin-coated microtiterplate (SA-MTP) in the first step. Not conjugated (unbound) antibody was removed by washing with universal buffer. Afterwards the samples and the reference standards (polyclonal rabbit anti-mAB IL-6R antibody spiked in 5% human serum) have been incubated in the wells. Anti-mAB IL-6R antibody bound to the immobilized mAB IL-6R. After having washed away unbound substances the bound anti-mAB IL-6R antibody was detected with digoxigenylated mAB IL-6R followed by incubation with a horse-radish peroxidase labeled anti-digoxigenin-antibody (see Figure 1). The antibody-enzyme conjugate catalyzed the color reaction of the ABTS^{®} substrate. The signal was measured by ELISA reader at 405 nm (reference wavelength: 490 nm). Absorbance values of each serum sample were determined in triplicate.

Five different variants of the bridging ELISA have been performed:
- variant 1 using conjugates of example 1 and 4
- variant 2 using conjugates of example 2 and 5
- variant 3 using conjugates of example 3 and 6
- variant 4 using mixed conjugates of examples 1 and 3 and mixed conjugates of examples 4 and 6
- variant 5 using mixed conjugates of examples 1-3 and mixed conjugates of examples 4-6.

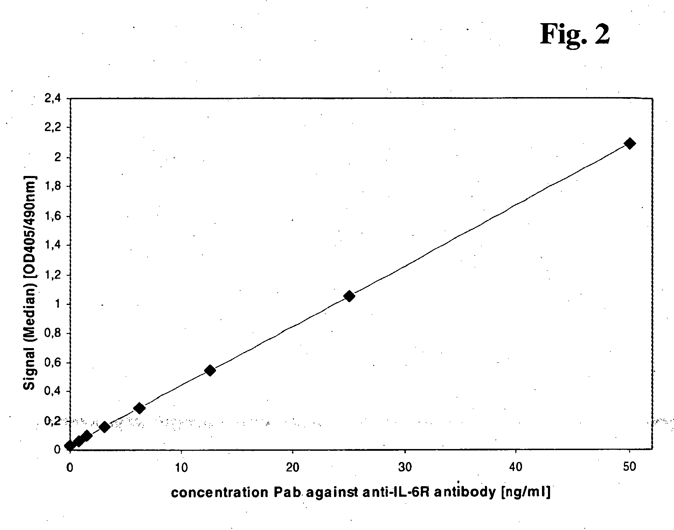

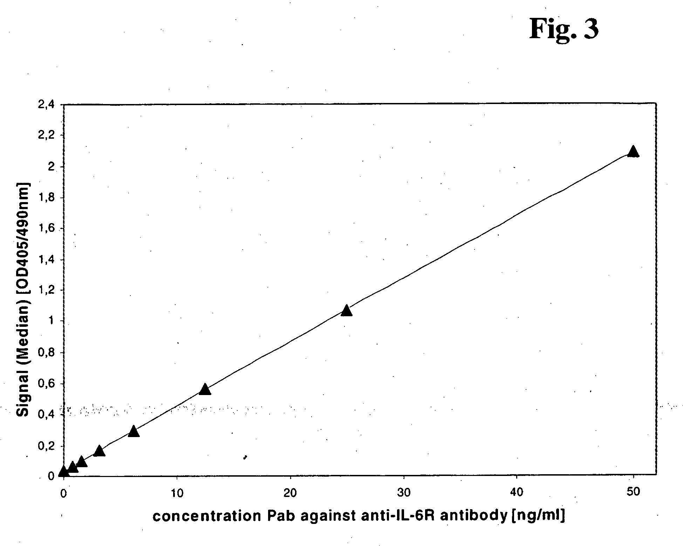

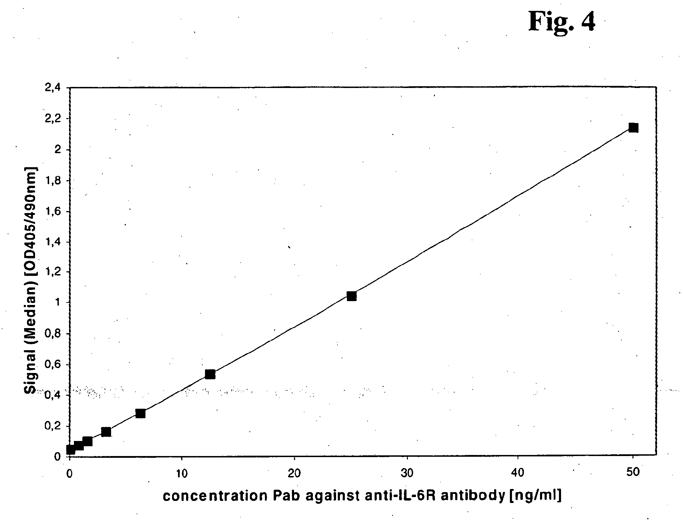

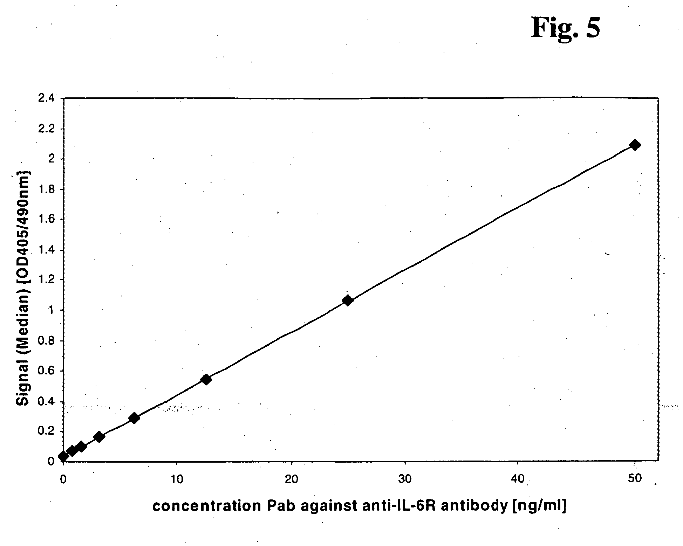

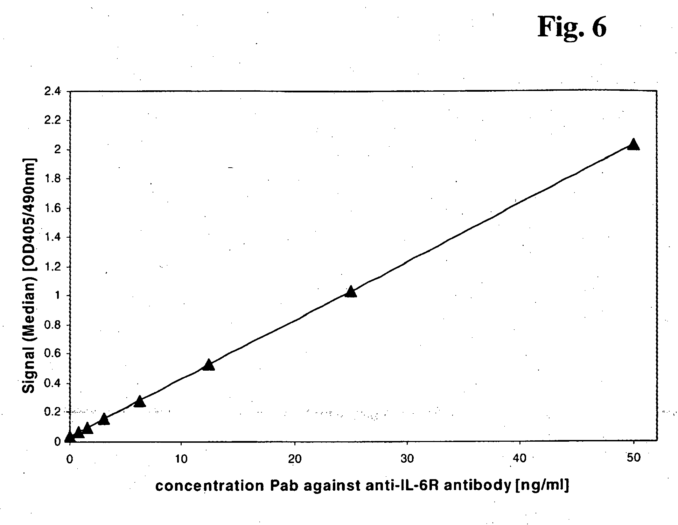

Reference standard signals and curves obtained in the different ELISA variants are shown in Table 1 and Figures 2-6.

**Table 1: Reference standard signals in the different ELISA variants.**

| **ref. conc. [ng/ml]** | **Signal variant 1** | **Signal variant 2** | **Signal variant 3** | **Signal variant 4** | **Signal variant 5** |
|---|---|---|---|---|---|
| 0.00 | 0.031 | 0.033 | 0.048 | 0.039 | 0.039 |
| 0.78 | 0.064 | 0.066 | 0.077 | 0.072 | 0.068 |
| 1.56 | 0.097 | 0.102 | 0.106 | 0.102 | 0.097 |
| 3.13 | 0.162 | 0.168 | 0.166 | 0.164 | 0.160 |
| 6.25 | 0.288 | 0.295 | 0.287 | 0.287 | 0.279 |
| 12.50 | 0.545 | 0.567 | 0.538 | 0.544 | 0.529 |
| 25.00 | 1.055 | 1.069 | 1.048 | 1.065 | 1.035 |
| 50.00 | 2.092 | 2.087 | 2.140 | 2.085 | 2.030 |

Sample analysis with the different standard curves is shown in Table 2.

**Table 2: Serum Sample analysis.**

| | **concentration pAB anti-mAB IL-6R equivalents [ng/ml]** | | | | |
|---|---|---|---|---|---|
| **Sample-Id** | **variant 1** | **variant 2** | **variant 3** | **variant 4** | **variant 5** |
| F262760-16 | 54.46 | 53.78 | 88.02 | 76.26 | 68.50 |
| F825050-26 | 10.21 | 11.51 | 4.57 | 7.88 | 9.71 |
| F963840-22 | 21.92 | 23.04 | 23.53 | 23.63 | 24.82 |
| E597480-16 | 76.02 | 76.29 | 55.18 | N/A | N/A |

As Table 1 shows, all conjugates can be used for detection of anti-mAB IL-6R antibodies. Using the same rabbit polyclonal anti-mAB IL-6R antibody the reference standard curves for all assay variants are very similar (Figures 2-6).

### Example 8

### Bridging ELISA for detection of anti-mAB IGF-1R antibodies using streptavidin/biotin interaction for immobilization at solid phase

Biotinylated antibody against IGF-1R (mAB IGF-1R, drug antibody) has been conjugated to (bound onto) the wells of a streptavidin-coated microtiterplate (SA-MTP) in the first step. Unconjugated (unbound) antibody was removed by washing with universal buffer. Afterwards the samples and the reference standards (polyclonal rabbit anti-mAB IGF-1R antibody spiked in 5% human serum) have been incubated in the wells. Anti-mAB IGF-1R antibody bound to the immobilized mAB IGF-1R. After having washed away unbound substances the bound anti-mAB IGF-1R antibody has been detected with digoxigenylated mAB IGF-1R followed by incubation with a horse-radish peroxidase labeled anti-digoxigenin-antibody. The antibody-enzyme conjugate catalyzed the color reaction of the ABTS^{®} substrate. The signal was measured by ELISA reader at 405 nm wavelength (reference wavelength: 490 nm). Absorbance values of each serum sample were determined in triplicates.

Three different variants of the bridging ELISA have been performed:
- variant 1 using conjugates made according to example 1 and 4
- variant 2 using conjugates made according to example 2 and 5
- variant 3 using mixed conjugates made according to examples 1 and 2 and mixed conjugates made according to examples 4 and 5.

All reagent variants of biotinylated and digoxygenylated mAB IGF-1R have been synthesized as described above for mAB IL-6R (examples 1, 2, 4 and 5).

Reference standard signals obtained in the different ELISA variants are shown in Table 3.

**Table 3: Reference standard signals in the different ELISA variants.**

| **ref. conc. [ng/ml]** | **Signal variant 1** | **Signal variant 2** | **Signal variant 3** |
|---|---|---|---|
| 0.00 | 0.126 | 0.056 | 0.110 |
| 0.31 | 0.161 | 0.092 | 0.147 |
| 0.63 | 0.205 | 0.131 | 0.191 |
| 1.25 | 0.286 | 0.203 | 0.267 |
| 2.50 | 0.440 | 0.348 | 0.425 |
| 5.00 | 0.772 | 0.660 | 0.744 |
| 10.00 | 1.349 | 1.223 | 1.321 |
| 20.00 | 2.113 | 2.060 | 2.133 |

Sample analysis with the different standard curves is shown in Table 4.

**Table 4: Serum sample analysis.**

| | **concentration pAB anti-mAB IGF-1R equivalents [ng/ml]** | | |
|---|---|---|---|
| **Sample-Id** | **variant 1** | **variant 2** | **variant 3** |
| 840 h_female | 2.04 | 10.64 | 7.27 |
| 1008 h_female | 11.38 | 24.05 | 14.05 |
| 504 h_male | 51.17 | 67.76 | 60.49 |

Table 3 shows that all conjugates can be used for detection of anti-mAB IGF-1R antibodies. Using the same rabbit polyclonal anti-mAB IGF-1R antibody the reference standard values for all assay variants are very similar (Table 3).

### Example 9

### Bridging-ELISA for detection of anti-mAB IGF-1R antibodies using passive adsorption for conjugation (immobilization) at a solid phase

A microtiter plate (MTP) (Maxisorb^{®}, Nunc) has been coated with mAB IGF-1R in carbonate buffer (pH 9.6), at room temperature (RT) for 1 hour. After washing three times with PBS-Tween^{®}20, all wells of the MTPs were blocked with PBS/3% (w/v) BSA (bovine serum albumine) at room temperature for 1 hour and then washed again. Afterwards the samples and the reference standards (polyclonal rabbit anti-mAB IGF-1R antibody spiked in 5% human serum) have been incubated. Anti-mAB IGF-1R antibody bound to the immobilized mAB IGF-1R. After having washed away unbound substances the bound anti-mAB IGF-1R antibody has been detected with digoxigenylated mAB IGF-1R followed by incubation with a horse-radish peroxidase labeled anti-digoxigenin-antibody. The antibody-enzyme conjugate catalyzed the color reaction of the ABTS^{®} substrate. The signal has been measured by ELISA reader at 405 nm wavelength (reference wavelength: 490 nm). Optical densities of each serum sample have been determined in triplicates.

Three different variants of the bridging ELISA have been performed:
- variant 1 using conjugates made according to example 4
- variant 2 using conjugates made according to example 5
- variant 3 using mixed conjugates made according to examples 4 and 5.

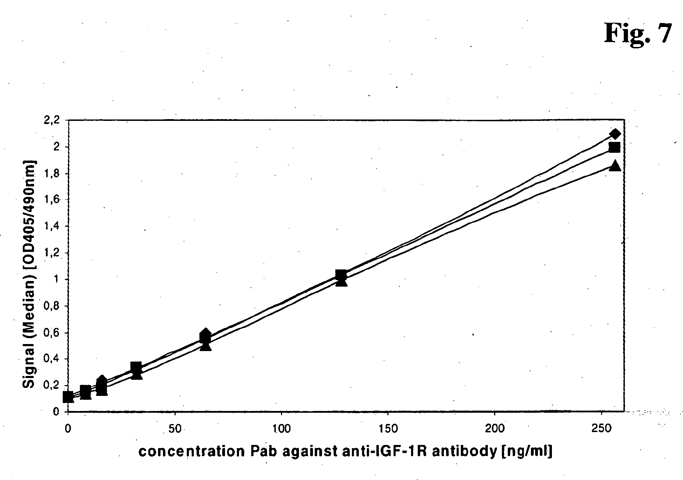

All reagent variants of digoxigenylated mAB IGF-1R have been synthesized as described above for mAB IL-6R (examples 4 and 5). Reference standard signals in the different ELISA variants are shown in Table 5 and Figure 7.

**Table 5: Reference standard signals.**

| **ref. conc. [ng/ml]** | **Signal variant 1** | **Signal variant 2** | **Signal variant 3** |
|---|---|---|---|
| 0.00 | 0.124 | 0.113 | 0.113 |
| 8.00 | 0.163 | 0.138 | 0.158 |
| 16.00 | 0.238 | 0.166 | 0.189 |
| 32.00 | 0.305 | 0.289 | 0.337 |
| 64.00 | 0.598 | 0.510 | 0.558 |
| 128.00 | 1.023 | 0.990 | 1.032 |
| 256.00 | 2.097 | 1.864 | 1.990 |

As Table 5 shows the bridging assay according to the invention using passive adsorption for conjugation (immobilization) of mAB IGF-1R on the solid phase can be conducted for detection of anti-mAB IGF-1R antibodies. Using the same rabbit polyclonal anti-mAB IGF-1R antibody the reference standard values for all three assay variants are very similar (Table 5).

## Claims

1. Method for the immunological determination of an antibody against a drug antibody in a sample using a double antigen bridging immunoassay comprising a capture drug antibody and a tracer drug antibody, **characterized in that**
i) the capture drug antibody is a mixture of said drug antibody comprising at least two of said drug antibodies that differ in the antibody site at which they are conjugated to the solid phase; and
ii) the tracer drug antibody is a mixture of said drug antibody comprising at least two of said drug antibodies that differ in the antibody site at which they are conjugated to the detectable label.

2. Method according to claim 1, **characterized in that** conjugation of the drug antibody to its conjugation partner is performed by chemically binding via N-terminal and/or ε-amino groups (lysine), ε-amino groups of different lysines, carboxy-, sulfhydryl-, hydroxyl- and/or phenolic functional groups of the amino acid backbone of the drug antibody and/or sugar alcohol groups of the carbohydrate structure of the drug antibody.

3. Method according to claim 1 or 2, **characterized in that** the tracer drug antibody mixture comprises the drug antibody conjugated via an amino group and via a carbohydrate structure to their conjugation partner.

4. Method according to any one of claims 1 to 3, **characterized in that** the capture drug antibody mixture comprises the drug antibody conjugated via an amino group and via a carbohydrate structure to their conjugation partner.

5. Method according to any one of claims 1 to 3, **characterized in that** conjugation of the capture drug antibody to the solid phase is performed by passive adsorption.

6. Method according to any one of claims 1 to 5, **characterized in that** the ratio of capture drug antibody to tracer drug antibody is 1:10 to 50:1 (ratio means ratio of antibody molecules irrespective of the molecular weight of the conjugates which can be different).

7. Method according to any one of claims 1 to 6, **characterized in that** the ratio of amino conjugated drug antibody (either tracer or capture drug antibody) to carbohydrate conjugated drug antibody (either tracer or capture drug antibody) in such a mixture is 1:10 to 10:1 (ratio means ratio of antibody molecules irrespective of the molecular weight of the conjugates which can be different).

8. Method according to any one of claims 1 to 4 and 6 to 7, **characterized in that** the capture drug antibody is immobilized via a specific binding pair.

9. Method according to claim 8, **characterized in that** the capture drug antibody is conjugated to biotin and immobilization is performed via immobilized avidin or streptavidin.

10. Method according to any one of claims 1 to 9, **characterized in that** the tracer drug antibody is conjugated to the detectable label via a specific binding pair.

11. Method according to claim 10, **characterized in that** the tracer drug antibody is conjugated to digoxigenin and linking to the detectable label is performed via an antibody against digoxigenin.

## Patentansprüche

1. Verfahren zur immunologischen Bestimmung eines Antikörpers gegen einen Arzneistoffantikörper in einer Probe unter Verwendung eines Doppel-Antigen-Brücken-Immunassays, umfassend einen Fänger-Arzneistoff-Antikörper und einen Tracer-Arzneistoff-Antikörper, **dadurch gekennzeichnet, dass**
i) der Fänger-Arzneistoff-Antikörper ein Gemisch aus dem Arzneistoff-Antikörper ist, der mindestens zwei der Arzneistoff-Antikörper umfasst, die sich an der Antikörperstelle, an der sie an die Festphase konjugiert sind, unterscheiden; und
ii) der Tracer-Arzneistoff-Antikörper ein Gemisch aus dem Arzneistoff-Antikörper ist, der mindestens zwei der Arzneistoff-Antikörper umfasst, die sich an der Antikörperstelle, an der sie an die nachweisbare Markierung konjugiert sind, unterscheiden.

2. Verfahren nach Anspruch 1, **dadurch gekennzeichnet, dass** die Konjugation des Arzneistoff-Antikörpers an seinen Konjugatpartner mittels einer chemischen Bindung über N-terminale Gruppen und/oder ε-Aminogruppen (Lysin), ε-Aminogruppen verschiedener Lysine, funktionelle Carboxy-, Sulfhydril-, Hydroxyl- und/oder Phenolgruppen des Aminosäurerückgrats des Arzneistoff-Antikörpers und/oder Zucker-Alkohol-Gruppen der Carbohydratstruktur des Arzneistoff-Antikörpers erfolgt.

3. Verfahren nach Anspruch 1 oder 2, **dadurch gekennzeichnet, dass** das Tracer-Arzneistoff-Antikörper-Gemisch den Arzneistoff-Antikörper über eine Aminogruppe und über eine Carbohydratstruktur an seinen Konjugatpartner konjugiert umfasst.

4. Verfahren nach einem der Ansprüche 1 bis 3, **dadurch gekennzeichnet, dass** das Fänger-Arzneistoff-Antikörper-Gemisch den Arzneistoff-Antikörper über eine Aminogruppe und über eine Carbohydratstruktur an seinen Konjugatpartner konjugiert umfasst.

5. Verfahren nach einem der Ansprüche 1 bis 3, **dadurch gekennzeichnet, dass** die Konjugation des Fänger-Arzneistoff-Antikörpers an die Festphase mittels passiver Adsorption erfolgt.

6. Verfahren nach einem der Ansprüche 1 bis 5, **dadurch gekennzeichnet, dass** das Verhältnis von Fänger-Arzneistoff-Antikörper zu Tracer-Arzneistoff-Antikörper 1: 10 bis 50: 1 ist (Verhältnis bedeutet das Verhältnis von Antikörpermolekülen unabhängig vom Molekulargewicht der Konjugate, das unterschiedlich sein kann).

7. Verfahren nach einem der Ansprüche 1 bis 6, **dadurch gekennzeichnet, dass** das Verhältnis von Amino-konjugiertem Arzneistoff-Antikörper (entweder Tracer-Arzneistoff-Antikörper oder Fänger-Arzneistoff-Antikörper) zu Carbohydrat-konjugiertem Arzneistoff-Antikörper (entweder Tracer-Arzneistoff-Antikörper oder Fänger-Arzneistoff-Antikörper) in einem solchen Gemisch 1: 10 bis 10:1 ist (Verhältnis bedeutet das Verhältnis von Antikörpermolekülen unabhängig vom Molekulargewicht der Konjugate, das unterschiedlich sein kann).

8. Verfahren nach einem der Ansprüche 1 bis 4 und 6 bis 7, **dadurch gekennzeichnet, dass** der Fänger-Arzneistoff-Antikörper über ein spezifisches Bindungspaar immobilisiert wird.

9. Verfahren nach Anspruch 8, **dadurch gekennzeichnet, dass** der Fänger-Arzneistoff-Antikörper an Biotin konjugiert ist und die Immobilisierung über immobilisiertes Avidin oder Streptavidin erfolgt.

10. Verfahren nach einem der Ansprüche 1 bis 9, **dadurch gekennzeichnet, dass** der Tracer-Arzneistoff-Antikörper über ein spezifisches Bindungspaar an die nachweisbare Markierung konjugiert ist.

11. Verfahren nach Anspruch 10, **dadurch gekennzeichnet, dass** der Tracer-Arzneistoff-Antikörper an Digoxigenin konjugiert ist und die Bindung an die nachweisbare Markierung über einen Antikörper gegen Digoxigenin erfolgt.

## Revendications

1. Procédé de détermination immunologique d'un anticorps dirigé contre un anticorps médicament dans un échantillon, utilisant une analyse immunologique avec double pontage antigénique comprenant un anticorps médicament de capture et un anticorps médicament traceur, **caractérisé en ce que**
i) l'anticorps médicament de capture est un mélange dudit anticorps médicament comprenant au moins deux desdits anticorps médicaments qui diffèrent par le site d'anticorps au niveau duquel ils sont conjugués à la phase solide ; et
ii) l'anticorps médicament traceur est un mélange dudit anticorps médicament comprenant au moins deux desdits anticorps médicaments qui diffèrent par le site d'anticorps au niveau duquel ils sont conjugués au marqueur détectable.

2. Procédé selon la revendication 1, **caractérisé en ce que** la conjugaison de l'anticorps médicament à son partenaire de conjugaison s'effectue par liaison chimique par l'intermédiaire de groupes N-terminaux et/ou ε-amino (lysine), de groupes ε-amino de différentes lysines, de groupes fonctionnels carboxy, sulfhydryle, hydroxyle et/ou phénoliques du squelette d'aminoacides de l'anticorps médicament et/ou de groupes alcool de sucre de la structure d'hydrates de carbone de l'anticorps médicament.

3. Procédé selon la revendication 1 à 2, **caractérisé en ce que** le mélange d'anticorps médicaments traceurs comprend l'anticorps médicament conjugué par l'intermédiaire d'un groupe amino et par l'intermédiaire d'une structure d'hydrates de carbone à leur partenaire de conjugaison.

4. Procédé selon l'une quelconque des revendications 1 à 3, **caractérisé en ce que** le mélange d'anticorps médicaments de capture comprend l'anticorps médicament conjugué par l'intermédiaire d'un groupe amino et par l'intermédiaire d'une structure d'hydrates de carbone à leur partenaire de conjugaison.

5. Procédé selon l'une quelconque des revendications 1 à 3, **caractérisé en ce que** la conjugaison de l'anticorps médicament de capture à la phase solide s'effectue par adsorption passive.

6. Procédé selon l'une quelconque des revendications 1 à 5, **caractérisé en ce que** le rapport de l'anticorps médicament de capture à l'anticorps médicament traceur est 1:10 à 50:1 (le rapport représente le rapport des molécules d'anticorps quelle que soit la masse moléculaire des conjugués qui peut être différente).

7. Procédé selon l'une quelconque des revendications 1 à 6, **caractérisé en ce que** le rapport de l'anticorps médicament conjugué par un amino (que ce soit l'anticorps médicament traceur ou de capture) à l'anticorps médicament conjugué par un hydrate de carbone (que ce soit l'anticorps médicament traceur ou de capture) dans un tel mélange est de 1:10 à 10:1 1 (le rapport représente le rapport des molécules d'anticorps quelle que soit la masse moléculaire des conjugués qui peut être différente).

8. Procédé selon l'une quelconque des revendications 1 à 4 et 6 à 7, **caractérisé en ce que** l'anticorps médicament de capture est immobilisé par l'intermédiaire d'une paire de liaison spécifique.

9. Procédé selon la revendication 8, **caractérisé en ce que** l'anticorps médicament de capture est conjugué à de la biotine et l'immobilisation s'effectue par l'intermédiaire d'avidine ou de streptavidine immobilisée.

10. Procédé selon l'une quelconque des revendications 1 à 9, **caractérisé en ce que** l'anticorps médicament traceur est conjugué au marqueur détectable par l'intermédiaire d'une paire de liaison spécifique.

11. Procédé selon la revendication 10, **caractérisé en ce que** l'anticorps médicament traceur est conjugué à de la digoxigénine et la liaison au marqueur détectable s'effectue par l'intermédiaire d'un anticorps dirigé contre la digoxigénine.
